# EUROPEAN PATENT SPECIFICATION

(11) **EP 2 735 305 B1**
(45) Date of publication and mention of the grant of the patent: **01.09.2021**
(21) Application number: 13194298.9
(22) Date of filing: 25.11.2013
(51) Int. Cl.: A61K 9/08, A61K 9/00, A61K 47/08, A61K 47/10, A61K 47/69, A61K 47/40, A61K 31/573, A61P 29/00

(54) **Stabilised liquid pharmaceutical preparations**
Stabilisierte flüssige pharmazeutische Zubereitungen
Préparations pharmaceutiques liquides stabilisées

(30) Priority: 26.11.2012 IT MI20122002
(43) Date of publication of application: 28.05.2014
(73) Proprietor: Laboratorio Farmacologico Milanese S.r.l., 21042 Caronno Pertusella (VA) (IT)
(72) Inventor: Valenti, Mauro, 20013 Magenta (MI) (IT)
(74) Representative: Bertuccio, Silvia

(56) References cited:
- EP-A1- 1 512 404
- WO-A1-99/36055
- WO-A1-2010/054356
- WO-A1-2010/128983
- WO-A2-2010/104584
- US-A1- 2006 046 970
- US-A1- 2009 137 539
- None

## Description

### TECHNICAL FIELD OF INVENTION

The present invention relates to stabilised liquid pharmaceutical preparations comprising dexamethasone sodium phosphate.

### STATE OF THE ART

Dexamethasone is a synthetic corticosteroid which is functionally analogous to the endogenous hormones cortisol and cortisone, but characterised by more specific pharmacokinetic and therapeutic properties and more modest side effects.

In particular, dexamethasone sodium phosphate presents from the chemical-physical standpoint as a white or whitish odourless crystalline powder with a bitter flavour, which is very hygroscopic and has a solubility in water of about 50 mg per ml. The pH of the aqueous solutions ranges from 7 to 8.5.

Dexamethasone sodium phosphate has the following structural formula:

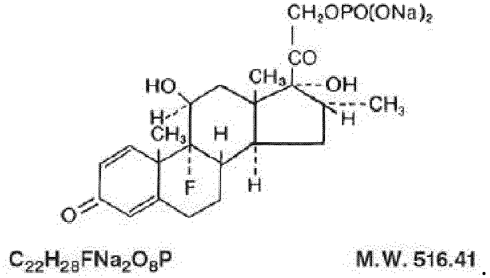

In the solid state it is relatively stable, but must be stored in tightly closed containers at a temperature from 2°C to 8°C.

Betamethasone is a synthetic corticosteroid with strong anti-inflammatory and anti-reactive activity, amounting to about 8-10 times that of prednisolone.

Betamethasone has a low tendency to cause the side effects characteristic of corticosteroids.

Betamethasone sodium phosphate has the following structural formula:

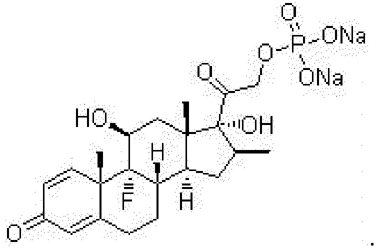

It presents in the form of a white, odourless powder which is highly soluble in water and poorly soluble in ethanol, and in aqueous solution has a pH ranging from 7 to 9.

The stability of said two corticosteroids is very low in aqueous solution, slightly better in glycol solvents, and strongly influenced by pH and storage temperature.

In general, they are indicated in the treatment of all inflammatory and allergic disorders requiring hormonal treatment based on corticosteroid hormones.

In particular, they are used to treat particularly intense allergic symptoms, rheumatic, dermatological and ophthalmological diseases, endocrine disorders of the adrenal glands, disorders of the respiratory system, haematological and neoplastic disorders.

The use of said molecules in treatment is justified by their strong anti-inflammatory activity, performed by inducing the enzyme lipocortin, which inhibits phospholipase A2 and the downstream cascade that leads, through lipoxygenase enzymes and the oxygenase cycle, to the production of inflammatory mediators and to antiallergic activity, probably due to the ability of dexamethasone to inhibit protein synthesis, leading to a significant reduction in the production of antibodies and the number of lymphocytes, eosinophils and basophils.

Their rapid gastrointestinal absorption and the corresponding biodistribution, mainly concentrated at vascular and platelet level, makes treatment with these corticosteroids particularly easy and very effective, although they (especially dexamethasone) are responsible for a number of side effects associated with their complex biological action mechanism.

The numerous formulations on the market containing dexamethasone or betamethasone in the form of a salt, such as sodium phosphate, increase their potential uses and the corresponding doses.

There are numerous pharmaceutical forms on the market which make them suitable for the treatment of more or less intense inflammatory symptoms.

For the treatment of the acute and more intense forms, there are a series of proprietary medicinal products in the form of injectable solutions, such as an injectable solution of dexamethasone sodium phosphate or betamethasone sodium phosphate in 4 mg/ml and 8 mg/2 ml ampoules.

A form consisting of oral drops containing 200 mg of dexamethasone sodium phosphate per 100 ml of solution is available for clinical symptoms requiring maintenance treatment and for particularly intense symptoms requiring high doses by repeated daily administrations.

Finally, an ointment containing 200 mg of dexamethasone sodium phosphate per 100 gm of ointment is available for topical treatment.

The following pharmaceutical forms of betamethasone are available on the market: injectable solution containing 400 mg/100 ml of betamethasone sodium phosphate, ophthalmic drops, oral drops containing 50 mg/100 ml of betamethasone, and tablets.

The use of said pharmaceutical forms is limited by problems associated with the comparatively low stability of the active ingredient in aqueous solutions, which requires a number of precautions to be taken for the correct storage of the various pharmaceutical forms.

The extreme sensitivity of these two corticosteroids to oxidation reduces the shelf life of the preparations, especially those in oral liquid form. The latter are also those mainly employed, due to their ease of use by elderly patients and to the possibility of devising tailored dose regimens suited to each patient, for example where doses much higher than those contained in the pre-determined pharmaceutical forms need to be taken.

In practice, nearly all the liquid preparations use solvents other than water, such as propylene glycol, as carriers.

However, propylene glycol, a solvent extensively used in the pharmaceutical field, has some contraindications concerning its relative liver toxicity compared with completely water-based or polyalcohol-based carriers and to its low palatability, which gives an unpleasant taste to preparations containing it. On the other hand, in many cases it provides better stability in solution of molecules which easily deteriorate in totally aqueous solvents.

However, the formation of a series of degradation products has even been observed under these conditions, which means that long, commercially advantageous shelf lives cannot be attributed to the formulations.

Stability studies conducted on aqueous or water-glycol solutions containing, for example, dexamethasone sodium phosphate have led to the identification of a series of degradation products, including:
- 3-oxo-9α-fluoro-11β-hydroxy-16α-methyl-17α-hydroxy-17β-carboxy-Δ^{1,4}-androstadiene; obtainable by hydrolysis of the phospho group and subsequent oxidation in C17.
- 9α-fluoro-11β-hydroxy-16α-methyl-androstan-1,4-diene-3,17-dione (C₂0H₂₅FO₃) (called DEXA A); obtainable by oxidation of dexamethasone formed in solution by hydrolysis of the phosphate group.
- 9α-fluoro-11β-hydroxy-14α-methyl-androstan-1,4-diene-3,17-dione (C₂0H₂₅FO₃) (called DEXA B); formed by isomerisation of DEXA A by migration of methyl from C15 to C14.
- 9α-fluoro-11β-17,21-trihydroxy-16α-methyl-pregna-1,4-diene-3,20-dione-17-(dihydrogen phosphate) disodium salt (C₂₂H₂₈FNA₂O₈P) (called DEX I).

This is the isomer of dexamethasone sodium phosphate, and is obtained by migration of the dihydrogen phosphate side chain.

The preparations currently on the market have a short shelf life and restrictive storage conditions: at most 25°C for 24 months.

US 2006/046970 discloses a topical otic composition containing an azalide antibiotic, a topical otic composition containing an azalide antibiotic and an medicament, and a topical otic composition containing an azalide antibiotic and a polymer suspending agent. It also discloses methods for treating or preventing infections in the ear using azalide antibiotic compositions.

WO 2010/104584 relates to articles and methods of treating vascular conditions with a thixotropic, turbid, bioactive agent-containing gel material capable of being essentially removed from an implantation site upon reestablishment of fluid flow at the implantation site.

EP 1 512 404 discloses an external medicine for treating dermatitis, wherein an adrenocortical steroid is included by a cyclodextrin; and 0.025 to 0.5% by weight of the adrenocortical steroid, 0.2 to 30% by weight of the cyclodextrin, and 0.5 to 55% by weight of dextran or pullulan are dissolved in an aqueous solution containing polysaccharide; and 0.5 to 55% by weight of xyloglucan, trehalose, laminaran, krestin, and pectin are blended.

There is consequently a need for liquid preparations containing dexamethasone salts, which possess better stability than the preparations present on the market, preferably in aqueous carriers, which are easily measured and more palatable, so that they are also suitable for oral administration.

### SUMMARY OF THE INVENTION

The present invention relates to preparations in liquid form containing dexamethasone sodium phosphate in a solubilizing aqueous system comprising propylene glycol in combination with cyclodextrins as stabilizing agents, and having a pH ranging from 7 to 8.

The invention also relates to these preparations for use in the prevention and/or treatment of inflammatory disorders.

### DESCRIPTION OF THE INVENTION

It has surprisingly been found that it is possible to obtain a preparation in stable liquid form comprising dexamethasone sodium phosphate by carrying the active constituent in a solubilizing system comprising an aqueous solution of propylene glycol in combination with cyclodextrins selected from the group consisting of beta-cyclodextrin, methyl-betacyclodextrin and hydroxypropylbetacyclodextrin as stabilizing agents.

The preparations have an aqueous base; the pH is from 7 to 8 if measured on the undiluted solutions, and preferably ranges from 7.1 to 7.3 if measured on solutions 20% diluted with purified water.

The solutions must be buffered to a pH ranging from 7 to 8 with suitable buffer systems of the phosphate or citrate type, or others able to maintain the pH within the indicated interval. For example, sodium hydroxide and sodium hydrogen phosphate dihydrate, or citric acid/sodium hydroxide buffer systems, can be used.

Aqueous solutions of propylene glycol can have a propylene glycol content ranging from 50 to 80%, more preferably from 65 to 75%.

The polyglycols, which can further be comprised in the preparations, can be polyethylene glycols selected from polyethylene glycol (PEG) 200, 300, 400, or mixtures thereof.

The polyalcohols, which can further be comprised in the preparations, are preferably sorbitol and glycerin.

The preparation can also contain polyoxyethylene hydroxystearate (solutol H15) and polyoxyl hydrogenated castor oil derivatives as solvents.

The stabilising agents are beta-cyclodextrin, methyl beta cyclodextrin or hydroxypropyl beta cyclodextrin; more preferably hydroxypropyl beta cyclodextrin.

In this case, the stabilising effect of the cyclodextrins may be due to their action against the most readily oxidisable functional groups of the active ingredient rather than to the classic inclusion action, which is mainly manifested as increased solubility of the active ingredient.

According to a preferred embodiment, the preparation in stable liquid form contains dexamethasone sodium phosphate in an aqueous solution of propylene glycol in combination with hydroxypropyl beta cyclodextrin and a buffer system able to maintain a pH from 7 to 8.

The aqueous solution can also include antioxidant agents.

The antioxidant agents, which protect the corticosteroid against oxidation, can be, for example, gallic acid and derivatives thereof (such as propyl gallate), ascorbic acid and derivatives thereof, tocopherols, compounds which have thiol functional groups (such as cystine, cysteine, acetyl cysteine, thiolactic acid, thioglycolic acid, glutathione, thiourea, methionine, etc.), sodium metabisulphite, etc.

The antioxidant action can also be synergised by the presence of antioxidant complexing agents, such as ethylenediaminetetraacetic acid (EDTA) and salts thereof, and by any compound structurally analogous with EDTA which exercises a protective action over the functional groups of the active ingredient.

Stabilisation can also be increased by inhibiting contact between the active ingredient and oxygen by manufacturing the preparation under constant bubbling in a nitrogen atmosphere.

According to a preferred embodiment, the preparation in stable liquid form contains dexamethasone sodium phosphate in an aqueous solution of propylene glycol in combination with hydroxypropyl beta cyclodextrin and disodium edetate and, by means of a buffer system, has a pH from 7 to 8.

According to a preferred aspect, dexamethasone or salts thereof, can be solubilised with suitable solubilising systems comprising glycol compounds such as aqueous solutions of propylene glycol and/or of polyethylene glycols (PEG) 200, 300 or 400 in combination with cyclodextrins and polyalcohols such as sorbitol and glycerin.

According to a further preferred aspect, the solubilising system comprises propylene glycol, purified water and polyethylene glycol (PEG) 400.

More preferably, when the preparation contains, for example, dexamethasone sodium phosphate ranging from 0.2 to 1% by weight, the amount of propylene glycol can range from 60 to 80% by weight and the amount of polyethylene glycol which, for example, can be PEG 400, can range from 1 to 5% by weight.

In particular, if a cyclodextrin is combined with propylene glycol and polyethylene glycol (PEG), the amounts of the various components can be, for example:
- propylene glycol 60-80% by weight,
- PEG 400 1-5% by weight,
- hydroxypropyl beta cyclodextrin 0.5-10% by weight,
- ethylenediaminetetraacetic acid and/or salts thereof 0.05-0.5% by weight,
- purified water, q.s. for 100%.

The preparations according to the invention can also be sweetened, for example, with sucrose, glucose, mannitol, fructose, laevulose or sweeteners used for sugar-free preparations, such as aspartame, saccharin, acesulfame, etc., or preferably with the addition of sodium saccharin in amounts ranging from 0.01 to 0.5% by weight, preferably from 0.1 to 0.2% by weight.

According to a further aspect of the invention, the preparations can include preservatives, such as sodium benzoate, benzoic acid, benzalkonium chloride, hydroxybenzoate and salts thereof, potassium sorbate and ascorbic acid.

The preparations thus obtained according to the invention surprisingly present far better stability than similar formulations on the market, and their toxicity is also much better than that of formulations containing propylene glycol alone, or products of the same chemical class.

Finally, the preparations according to the invention have improved palatability, which makes them more acceptable to children, the elderly and patients who need to take large doses (such as 2-3 ml/administration) to treat serious acute disorders.

The preparations according to the invention can be useful in the prevention and/or treatment of inflammatory disorders.

### EXAMPLES

The examples given below further illustrate the invention.

### EXAMPLE 1

### Preparation for oral solution (drops)

| | Components | Unit of measurement | Composition per 100 ml |
|---|---|---|---|
| 1 | Dexamethasone sodium phosphate | mg | 200 |
| 2 | Propylene glycol | g | 70.0 |
| 3 | Hydroxypropyl beta cyclodextrin | g | 0.65 |
| 4 | Sodium saccharin | g | 0.20 |
| 5 | Sodium benzoate | g | 0.15 |
| 6 | Disodium edetate | g | 0.10 |
| 7 | Sodium dihydrogen phosphate dihydrate | | q.s. for pH 7.0-8.0 |
| 8 | Sodium hydroxide | | q.s. for pH 7.0-8.0 |
| 9 | Purified water | | q.s. for 100 ml |

The preparation was produced according to the conventional techniques used for the manufacture of liquid oral solutions.

As all the components of the solution are completely soluble in water and propylene glycol, the process was entirely conducted at room temperature.

The salient steps of the manufacturing process are as follows:
- dissolution in part of the water amount of hydroxypropyl beta cyclodextrin and further solubilisation of the excipients, in particular disodium edetate, sodium benzoate, sodium saccharin and sodium dihydrogen phosphate dihydrate;
- subsequent dissolution of active ingredient dexamethasone sodium phosphate;
- addition of propylene glycol;
- control of pH by adding sodium hydroxide solution until a pH from 7 to 8 is obtained;
- addition of water to the final volume and retesting of pH;
- filtration.

### Vial-filling

The solution obtained was introduced into vials and subjected to a stability study (under accelerated and normal conditions according to a protocol complying with the requirements of the European Guideline, CPMP/ICH/2736/99 "Note for Guidance on Stability Testing: Stability Testing of New Drug Substances and Products") under the following conditions:
- accelerated conditions for 6 months: 40°C ± 2°C/75% RH ± 5% RH
- intermediate conditions for 12 months: 30°C ± 2°C/65% RH ± 5% RH
- room temperature for 36 months: 25°C ± 2°C/60% RH ± 5%

On the date specified for each condition the samples were analysed, with special reference to the formation of degradation products, with specific, stability-indicating, validated analysis methods.

For all storage conditions it was surprisingly found that the formation of degradation products was either not observed or, in some cases, was very slight, and in any event within the range tolerated by the ICH guidelines.

### EXAMPLE 2

**Preparation for oral solution**

| | Components | Unit of measurement | Composition per 100 ml |
|---|---|---|---|
| 1 | Dexamethasone sodium phosphate | mg | 200 |
| 2 | Propylene glycol | g | 60.0 |
| 3 | PEG 400 | g | 10.0 |
| 4 | Hydroxypropyl beta cyclodextrin | g | 0.65 |
| 5 | Sodium saccharin | g | 0.20 |
| 6 | Sodium benzoate | g | 0.15 |
| 7 | Disodium edetate | g | 0.10 |
| 8 | Sodium dihydrogen phosphate dihydrate | | q.s. for pH 7.0-8.0 |
| 9 | Sodium hydroxide | | q.s. for pH 7.0-8.0 |
| 10 | Purified water | | q.s. for 100 ml |

The procedure described in Example 1 was repeated, replacing part of propylene glycol with PEG 400.

### EXAMPLE 3

**Preparation for oral solution**

| | Components | Unit of measurement | Composition per 100 ml |
|---|---|---|---|
| 1 | Dexamethasone sodium phosphate | mg | 400 |
| 2 | Propylene glycol | g | 60.0 |
| 3 | PEG 400 | g | 10.0 |
| 4 | Hydroxypropyl beta cyclodextrin | g | 1.3 |
| 5 | Sodium saccharin | g | 0.20 |
| 6 | Sodium benzoate | g | 0.15 |
| 7 | Disodium edetate | g | 0.10 |
| 8 | Sodium dihydrogen phosphate dihydrate | | q.s. for pH 7.0-8.0 |
| 9 | Sodium hydroxide | | q.s. for pH 7.0-8.0 |
| 10 | Purified water | | q.s. for 100 ml |

The procedure described in Example 1 was repeated, replacing part of propylene glycol with PEG 400.

### EXAMPLE 4

**Preparation of an aqueous solution for injectable liquids**

| | Components | Unit of measurement | Composition per 100 ml |
|---|---|---|---|
| 1 | Dexamethasone sodium phosphate | mg | 200 |
| 2 | Propylene glycol | g | 10.0 |
| 3 | Hydroxypropyl beta cyclodextrin | g | 0.65 |
| 4 | Sodium dihydrogen phosphate dihydrate | | q.s. for pH 7.0-8.0 |
| 5 | Sodium hydroxide | | q.s. for pH 7.0-8.0 |
| 6 | Purified water | | q.s. for 100 ml |

The procedure is as described in Example 1.

### EXAMPLE 5

**Preparation of an aqueous solution for injectable liquids**

| | Components | Unit of measurement | Composition per 100 ml |
|---|---|---|---|
| 1 | Dexamethasone sodium phosphate | mg | 200 |
| 2 | Propylene glycol | g | 20.0 |
| 3 | Hydroxypropyl beta cyclodextrin | g | 0.65 |
| 4 | Sodium dihydrogen phosphate dihydrate | | q.s. for pH 7.0-8.0 |
| 5 | Sodium hydroxide | | q.s. for pH 7.0-8.0 |
| 6 | Purified water | | q.s. for 100 ml |

The preparation process is as described in Example 1.

### EXAMPLE 6

**Preparation in syrup**

| | Components | Unit of measurement | Composition per 100 ml |
|---|---|---|---|
| 1 | Dexamethasone sodium phosphate | mg | 20.0-40.0 |
| 2 | Propylene glycol | g | 40.0 |
| 3 | PEG 400 | g | 10.0 |
| 4 | Hydroxypropyl beta cyclodextrin | g | 0.65 |
| 5 | Sorbitol | g | 30.0 |
| 6 | Sodium benzoate | g | 0.15 |
| 7 | Disodium edetate | g | 0.10 |
| 8 | Sodium dihydrogen phosphate dihydrate | | q.s. for pH 7.0 |
| 9 | Sodium hydroxide | | q.s. for pH 7.0-8.0 |
| 10 | Purified water | | q.s. for 100 ml |

The preparation process is as described in Example 1.

### EXAMPLE 7

Formulation A according to the invention and Formulation B (not part of the invention), having the composition set out below, in the form of oral drops were prepared.

**FORMULATION A (according to the invention)**

| **INGREDIENTS** | **Quantity for 1 mL** | **Quantity for 30 Lt** |
|---|---|---|
| Dexamethasone sodium phosphate | 2.00 mg | 60.00 g |
| Sodium benzoate | 1.50 mg | 45.00 g |
| Propylene glycol | 700.00 mg | 21.00 Kg |
| Sodium dihydrogen Phosphate dihydrate | 5.50 mg | 165.00 g |
| Saccharin sodium | 2.00 mg | 60.00 g |
| Hydroxypropylbetadex | 6.50 mg | 195.00 g |
| Disodium edetate | 1.00 mg | 30.00 g |
| Sodium hydroxide | 0.6667 mg | 20.00 g |
| Purified water | q.s. to 1.00 mL | q.s. to 30.00 Lt |
| | (328 mg) | (9.84 Kg) |
| *Total weight** | *1046.50 mg* | *31.395 Kg* |

| | | |
|---|---|---|
| * *Relative Density (d*= *1.0465 g*/*mL)* | | |

**FORMULATION B (not part of the invention)**

| **INGREDIENTS** | **Quantity for 1 mL** | **Quantity for 30 Lt** |
|---|---|---|
| Dexamethasone sodium phosphate | 2.00 mg | 60.00 g |
| Propylene glycol | q.s. to 1.00 mL | q.s. to 30 Lt |
| *Total weight** | *1030.60 mg* | *31.08 Kg* |

| | | |
|---|---|---|
| * *Relative Density (d*= *1.036 g*/*mL)* | | |

### STABILITY TESTS

The stability of Formulation B has been tasted at T=25°C for 24 months for batch No. 010110 and at T=25°C for 36 months for batch No. 010109. The results are reported in the tables below.

| **STABILITY STUDY RESULTS BATCH No. 010109 STORAGECONDITIONS: 25°C±2°C/60%RH±5%** | | | | | |
|---|---|---|---|---|---|
| **TESTS** | **SPECIFICATIONS** | **T0** | **T12** | **T24** | **T36** |
| pH | 7,0 - 8,0 | 7,3 | 7,6 | 7,6 | 7,6 |
| Dexamethasone sodium phosphate assay | 95.0 - 105.0% | 98.9 | 100.0 | 96.1 | 98.2 |

| Impurities: | | | | | |
|---|---|---|---|---|---|
| Dexa 1 | <1.0% | <LOD | 0.06 | 0.11 | 0.10 |
| Dexa A | <1.0% | <LOD | 0.38 | 0.89 | 1.5 |
| Dexa B | <1.0% | <LOD | 0.17 | 0.51 | 1.1 |

| **STABILITY STUDY RESULTS BATCH No. 010110 STORAGE CONDITIONS: 25°C±2°C/60%RH±5%** | | | | |
|---|---|---|---|---|
| **TESTS** | **SPECIFICATIONS** | **T0** | **T12** | **T24** |
| pH | 7.0 - 8.0 | 7.6 | 7.6 | 7.8 |
| Dexamethasone sodium phosphate assay | 95.0 - 105.0% | 103.5 | 101.0 | 99.5 |

| Impurities: | | | | |
|---|---|---|---|---|
| Dexa 1 | <1.0% | <LOD | <LOQ | 0.05 |
| Dexa A | <1.0% | <LOD | 0.55 | 1.7 |
| Dexa B | <1.0% | <LOD | 0.28 | 1.0 |
| Dexa unk | <1.0% | <LOQ | 0.39 | 1.0 |

| | | | | |
|---|---|---|---|---|
| LOD=Limit Of Detection; LOQ=Limit Of Quantitation | | | | |

The stability data show the formation of degradation products of Dexamethasone, that are DEXA 1, DEXA A e DEXA B, in significat amounts and in any case in amounts higher than the acceptable fixed values.

The stability of Formulation A has been tested at T=25°C for 24 months and at T=30°C for 12 months for batch No. 010610 as well as for batch 020610 at T=25°C for 36 months and at T=30°C for 12 months.

The results are reported in the tables below 8 (LOD=Limit Of Detection; LOQ=Limit Of Quantitation).

| **STABILITY STUDY RESULTS BATCH No. 010610 STORAGE CONDITIONS: 25°C±2°C/65% RH±5%** | | | | | | | | |
|---|---|---|---|---|---|---|---|---|
| **TESTS** | **SPECIFICATIONS** | **T0** | **T3** | **T6** | **T9** | **T12** | **T18** | **T24** |
| pH | 7.0 - 7.5 | 7.1 | 7.0 | 7.2 | 7.1 | 7.1 | 7.1 | 7.0 |
| Dexamethasone sodium phosphate assay | 95.0 - 105.0% | 104.3 | 103.6 | 100.6 | 101 | 99.1 | 103.1 | 103.4 |
| Impurities | | | | | | | | |
| Imp. Dexa I | <0.4% | <LOD | <LOQ | <LOQ | <LOQ | <LOD | <LOQ | <LOQ |
| Imp. Dexa A | <0.4% | <LOQ | <LOD | <LOD | <LOD | <LOD | <LOQ | <LOD |
| Imp. Dexa B | <0.4% | <LOD | <LOD | <LOD | <LOD | <LOD | <LOD | <LOD |
| Imp. Dexa Unk. | ≤0.4% | <LOD | <LOQ | <LOD | <LOD | <LOD | <LOD | <LOD |

| **STABILITY STUDY RESULTS BATCH No. 010610 STORAGE CONDITIONS 30°C±2°C/65% RH±5%** | | | | | | |
|---|---|---|---|---|---|---|
| **TESTS** | **SPECIFICATIONS** | **T0** | **T3** | **T6** | **T9** | **T12** |
| pH | 7.0 - 7.5 | 7.1 | 7.0 | 7.1 | 7.1 | 7.1 |
| Dexamethasone sodium phosphate assay | 95.0 - 105.0% | 104.3 | 103.4 | 99.5 | 98.9 | 98.4 |
| Impurities | | | | | | |
| Imp. Dexa I | <0.4% | <LOD | <LOD | <LOQ | 0.07 | <LOQ |
| Imp. Dexa A | <0.4% | <LOQ | <LOD | <LOD | <LOD | <LOQ |
| Imp. Dexa B | <0.4% | <LOD | <LOD | <LOD | <LOD | <LOD |
| Imp. Dexa Unk. | <0.4% | <LOD | <LOQ | <LOD | <LOD | <LOD |

| **STABILITY STUDY RESULTS BATCH No. 020610 STORAGE CONDITIONS 25°C±2°C/65% RH±5%** | | | | | | | | | |
|---|---|---|---|---|---|---|---|---|---|
| **TESTS** | **SPECIFICATIONS** | **T0** | **T3** | **T6** | **T9** | **T12** | **T18** | **T24** | **T36** |
| pH | 7.0 - 7.5 | 7.1 | 7.0 | 7.2 | 7.1 | 7.1 | 7.1 | 7.0 | 7.1 |
| Dexamethasone sodium phosphate assay | 95.0 - 105.0% | 103.7 | 102.5 | 100.8 | 101.3 | 98.6 | 103.3 | 103.6 | 98.9 |
| Impurities | | | | | | | | | |
| Imp. Dexa I | ≤0.4% | <LOD | <LOQ | <LOD | <LOQ | <LOD | <LOQ | <LOQ | <LOQ |
| Imp. Dexa A | ≤0.4% | <LOD | <LOD | <LOD | <LOD | <LOD | <LOQ | <LOD | <LOD |
| Imp. Dexa B | ≤0.4% | <LOD | <LOD | <LOD | <LOD | <LOD | <LOD | <LOD | <LOD |
| Imp. Dexa Unk. | ≤0.4% | <LOQ | <LOD | <LOD | <LOD | <LOD | <LOD | <LOD | <LOQ |

| **STABILITY STUDY RESULTS BATCH No. 020610 STORAGE CONDITIONS: 30°C±2°C/65% RH±5%** | | | | | | |
|---|---|---|---|---|---|---|
| **TESTS** | **SPECIFICATIONS** | **T0** | **T3** | **T6** | **T9** | **T12** |
| pH | 7.0 - 7.5 | 7.1 | 7.0 | 7.1 | 7.1 | 7.1 |
| Dexamethasone sodium phosphate assay | 95.0 - 105.0% | 103.7 | 102.0 | 101.1 | 100.3 | 99.4 |
| Impurities | | | | | | |
| Imp. Dexa I | <0.4% | <LOD | <LOQ | <LOQ | 0.07 | <LOQ |
| Imp. Dexa A | <0.4% | <LOD | <LOD | <LOD | <LOD | <LOQ |
| Imp. Dexa B | <0.4% | <LOD | <LOD | <LOD | <LOD | <LOD |
| Imp. Dexa Unk. | <0.4% | <LOQ | 0.06 | <LOD | <LOD | <LOD |

The stability data show the degradation products of Dexamethasone, that are DEXA 1, DEXA A e DEXA B. The total sum of the degradation products has been drastically reduced from about 5% for Formulation B to less than 1% for Formulation A, i.e. to an acceptable value.

As to the formation of degradation products in samples stored at T= 30°C, it appears that the results are perfectly in line with those obtained for samples stored at T=25°C. This allows the new formulation to have a longer shelf life (36 months if stored at room temperature).

## Claims

1. Preparation in liquid form comprising dexamethasone sodium phosphate in a solubilizing aqueous system comprising propylene glycol in combination with cyclodextrins selected from the group consisting of beta-cyclodextrin, methylbetacyclodextrin and hydroxypropylbetacyclodextrin as stabilizing agents, and having a pH ranging from 7 to 8.

2. Preparation according to claim 1, further comprising polyethylene glycols selected from the group consisting of polyethylene glycol (PEG) 200, 300, 400, or mixtures thereof.

3. Preparation according to claim 1 or 2, further comprising polyalcohols selected from sorbitol and glycerin.

4. Preparation according to claims 1-3, wherein the cyclodextrin is hydroxypropylbetacyclodextrin.

5. Preparation according to claims 1-4, comprising dexamethasone sodium phosphate in an aqueous solution of propylene glycol in combination with hydroxypropylbetacyclodextrin, and having a pH ranging from 7 to 8.

6. Preparation according to claims 1-5, further comprising antioxidant agents.

7. Preparation according to claim 6, wherein the antioxidant agents are selected from the group consisting of ethylenediaminetetraacetic acid (EDTA) and salts or analogues thereof.

8. Preparation according to claims 1-7, comprising dexamethasone sodium phosphate in an aqueous solution of propylene glycol in combination with hydroxypropylbetacyclodextrin and disodium edetate buffered to a pH ranging from 7 to 8.

9. Preparation according to any one of the above claims 1-8, for use in the prevention and/or treatment of inflammatory disorders.

## Patentansprüche

1. Zubereitung in flüssiger Form, umfassend Dexamethason-Natriumphosphat in einem solubilisierenden wässrigen System, umfassend Propylenglycol in Kombination mit Cyclodextrinen, ausgewählt aus der Gruppe, bestehend aus beta-Cyclodextrin, Methylbetacyclodextrin und Hydroxypropylbetacyclodextrin, als stabilisierende Mittel, und mit einem pH-Wert im Bereich von 7 bis 8.

2. Zubereitung nach Anspruch 1, des Weiteren umfassend Polyethylenglycole, ausgewählt aus der Gruppe, bestehend aus Polyethylenglycol (PEG) 200, 300, 400 oder Gemischen davon.

3. Zubereitung nach Anspruch 1 oder 2, des Weiteren umfassend Polyalkohole, ausgewählt aus Sorbitol und Glycerin.

4. Zubereitung nach den Ansprüchen 1-3, wobei das Cyclodextrin Hydroxypropylbetacyclodextrin ist.

5. Zubereitung nach den Ansprüchen 1-4, umfassend Dexamethason-Natriumphosphat in einer wässrigen Lösung aus Propylenglycol in Kombination mit Hydroxypropylbetacyclodextrin und mit einem pH-Wert im Bereich von 7 bis 8.

6. Zubereitung nach den Ansprüchen 1-5, des Weiteren umfassend Antioxidationsmittel.

7. Zubereitung nach Anspruch 6, wobei die Antioxidationsmittel ausgewählt sind aus der Gruppe, bestehend aus Ethylendiamintetraessigsäure (EDTA) und Salzen oder Analoga davon.

8. Zubereitung nach den Ansprüchen 1-7, umfassend Dexamethason-Natriumphosphat in einer wässrigen Lösung aus Propylenglycol in Kombination mit Hydroxypropylbetacyclodextrin und Dinatriumedetat, gepuffert auf einen pH-Wert im Bereich von 7 bis 8.

9. Zubereitung nach einem der obigen Ansprüche 1-8 zur Verwendung bei der Prävention und/oder Behandlung von entzündlichen Erkrankungen.

## Revendications

1. Préparation sous la forme liquide comprenant du phosphate de dexaméthasone sodique dans un système de solubilisation aqueux comprenant du propylène glycol en combinaison avec des cyclodextrines sélectionnées dans le groupe constitué de la bêta-cyclodextrine, de la méthylbêtacyclodextrine et de l'hydroxypropylbêtacyclodextrine comme agents stabilisants, et ayant un pH s'étendant de 7 à 8.

2. Préparation selon la revendication 1, comprenant en outre des polyéthylènes glycols sélectionnés dans le groupe constitué du polyéthylène glycol (PEG) 200, 300, 400, ou leurs mélanges.

3. Préparation selon la revendication 1 ou 2, comprenant en outre des polyalcools sélectionnés parmi le sorbitol et la glycérine.

4. Préparation selon les revendications 1 à 3, la cyclodextrine étant l'hydroxypropylbêtacyclodextrine.

5. Préparation selon les revendications 1 à 4, comprenant du phosphate de dexaméthasone sodique dans une solution aqueuse de propylène glycol en combinaison avec de l'hydroxypropylbêtacyclodextrine, et ayant un pH s'étendant de 7 à 8.

6. Préparation selon les revendications 1 à 5, comprenant en outre des agents antioxydants.

7. Préparation selon la revendication 6, les agents antioxydants étant sélectionnés dans le groupe constitué de l'acide d'éthylènediaminetétraacétique (EDTA) et de ses sels ou analogues.

8. Préparation selon les revendications 1 à 7, comprenant du phosphate de dexaméthasone sodique dans une solution aqueuse de propylène glycol en combinaison avec de l'hydroxypropylbêtacyclodextrine et de l'édétate disodique tamponné à un pH s'étendant de 7 à 8.

9. Préparation selon l'une quelconque des revendications ci-dessus 1 à 8, pour l'utilisation dans la prévention et/ou le traitement des troubles inflammatoires.
